# EUROPÄISCHE PATENTANMELDUNG

(11) **EP 1 188 411 A1**
(43) Veröffentlichungstag der Anmeldung: **20.03.2002**
(21) Anmeldenummer: 01117786.2
(22) Anmeldetag: 02.08.2001
(51) Int. Cl.: A61B 3/12, A61B 3/15

(54) **Verfahren und Vorrichtung für eine Untersuchung des Augenhintergrundes eines Patienten**

(30) Priorität: 31.08.2000 DE 10042718
(71) Anmelder: Pro-Laser Weco GmbH, 40231 Düsseldorf (DE)
(72) Erfinder: Reis, Werner, 80992 München (DE); Spaltmann, Ronald, 83224 Grassau (DE)
(74) Vertreter: Cohausz & Florack

(57) **Zusammenfassung**

Die Erfindung betrifft ein Verfahren für eine Untersuchung des Augenhintergrundes eines Patienten in einem abgedunkelten Raum sowie ein Ophthalmoskop für die Durchführung dieses Verfahrens. Die Aufgabe, eine kontinuierliche Bildaufzeichnung des Augenhintergrundes bei gleichzeitig schonender Beleuchtung zu realisieren, wird dadurch gelöst, daß ein Beleuchtungsstrahl (4) auf den Augenhintergrund abgebildet wird, daß das vom Augenhintergrund als Beobachtungsstrahl (18) reflektierte Licht als ein reelles Zwischenbild abgebildet wird, daß das reelle Zwischenbild mit einer Beobachtungseinrichtung (22) aufgenommen wird, daß mittels eines Infrarotfilters (28) in einer ersten Einstellung der infrarote Lichtanteil des Beleuchtungsstrahls (4) zumindest teilweise bevorzugt durchgelassen und auf das Auge abgebildet wird und daß während einer zweiten Zeitdauer für eine Aufnahme des Augenhintergrundes mit sichtbarem Licht zumindest teilweise der sichtbare Lichtanteil des Beleuchtungsstrahls (4) bevorzugt durchgelassen und auf das Auge abgebildet wird.

## Beschreibung

Die Erfindung betrifft ein Verfahren für eine Untersuchung des Augenhintergrundes eines Patienten in einem abgedunkelten Raum sowie ein Ophthalmoskop für die Durchführung dieses Verfahrens.

Um das Anwendungsspektrum von Ophthalmoskopen für die Allgemeinmedizin, zum Beispiel bei Reihenuntersuchungen, und für Nichtmediziner, die keine Erlaubnis zur medikamentösen Pupillenerweiterung besitzen, zu erweitern, ist es erforderlich, die Pupille des zu untersuchenden Auges möglichst groß zu halten.

Mit einer normalen Beleuchtung im sichtbaren Bereich wird bei Lichteinfall auf die Iris die Pupille stark verkleinert. Die beiden geometrisch getrennt verlaufenden Strahlengänge des Beleuchtungsstrahls einerseits und des Beobachtungsstrahls andererseits können dann nicht optimal in das Auge abgebildet werden. Die ungewollten Augenbewegungen, speziell bei jungen und älteren Menschen spielen dabei einen großen Einfluß, so daß oftmals ein brauchbares, reproduzierbares Bild des Augenhintergrundes (Fundus) nicht ausreichend genau eingestellt werden kann.

Aus dem Stand der Technik ist es bekannt, in einem abgedunkelten Raum bei stark erweiterter Pupille des Auges mit einer separaten Beleuchtungsoptik, die elektromagnetische Strahlung im nahen Infrarotbereich emitiert und somit keine Blendung des Auges bewirkt, eine auch im Infrarotbereich empfindliche Kamera auf den Augenhintergrund einzustellen. Für eine Aufnahme des Augenhintergrundes wird dann eine weitere Beleuchtungsoptik aktiviert, die mittels Lichtblitzen Momentaufnahmen des Augenhintergrundes erzeugt. Dabei sind die Lichtblitze im sichtbaren Bereich so stark, daß sich die Augenpupille schnell verkleinert und der Beobachtungszeitraum für eine Abbildung des Augenhintergrundes stark verkürzt ist. Zudem muß vor der Aufnahme mit Hilfe eines Lichtblitzes die Kameraeinstellung aufgrund der erhöhten Lichtintensität verändert werden, um ein auswertbares Bild des Augenhintergrundes aufnehmen zu können.

Insgesamt ist es somit möglich, ohne den Einsatz von Medikamenten die natürliche Aufweitung der Pupille des zu untersuchenden Auges für Einzelaufnahmen des Augenhintergrundes auszunutzen. Aufgrund der Verwendung separater Beleuchtungseinrichtungen ist der technische Aufwand dabei recht groß. Zudem sind nur Momentaufnahmen wegen des Einsatzes von Lichtblitzen möglich, wobei das Patientenauge durch die starke Blitzlichteinstrahlung stark belastet wird.

Der Erfindung liegt daher das technische Problem zugrunde, ein Verfahren für eine Untersuchung des Augenhintergrundes eines Patienten in einem abgedunkelten Raum sowie ein dafür geeignetes Ophthalmoskop anzugeben, bei dem eine kontinuierliche Bildaufzeichnung des Augenhintergrundes bei gleichzeitig schonender Beleuchtung realisiert wird.

Das zuvor aufgezeigte technische Problem wird zunächst durch ein Verfahren gemäß Anspruch 1 gelöst, bei dem ein Beleuchtungsstrahl auf den Augenhintergrund abgebildet wird, bei dem das vom Augenhintergrund als Beobachtungsstrahl reflektierte Licht als ein reelles Zwischenbild abgebildet wird und bei dem das reelle Zwischenbild mit einer Beobachtungseinrichtung aufgenommen wird, bei dem während einer ersten Zeitdauer für eine Einstellung der Beobachtungseinrichtung der infrarote Lichtanteil des Beleuchtungsstrahls auf das Auge abgebildet wird und bei dem während einer zweiten Zeitdauer für eine Aufnahme des Augenhintergrundes mit sichtbarem Licht zumindest teilweise der sichtbare Lichtanteil des Beleuchtungsstrahls auf das Auge abgebildet wird.

Erfindungsgemäß ist somit erkannt worden, daß ein und die gleiche Beleuchtungseinrichtung verwendet werden kann, um einerseits einen im wesentlichen nur aus Infrarotstrahlung bestehenden Beleuchtungsstrahl und andererseits einen sichtbares Licht enthaltenden Beleuchtungsstrahl zu erzeugen. Somit kann während der ersten Zeitdauer, in der das Auge nur mit infrarotem, also nicht sichtbarem Licht bestrahlt wird und die Pupille des Auges dementsprechend aufgeweitet ist, die Kameraeinstellung bezüglich der Position des Augenhintergrundes als auch in Bezug auf die Scharfstellung eingestellt werden. Wird dann während der zweiten Zeitdauer sichtbares Licht von der gleichen Beleuchtungseinrichtung auf das Auge abgebildet, so ist eine Aufnahme des Augenhintergrundes im sichtbaren Bereich möglich. Da sich zwischen der ersten und zweiten Zeitdauer der Strahlengang nicht ändert, kann die während der ersten Zeitdauer eingestellte Kameraeinstellung beibehalten werden, um während der ersten Zeitdauer ein scharfes Abbild des Augenhintergrundes mit sichtbarem Licht zu erzeugen.

In bevorzugter Weise wird während der zweiten Zeitdauer der sichtbare Lichtanteil des Beleuchtungsstrahls bevorzugt durchgelassen. Der infrarote Strahlungsanteil wird somit stark vermindert, vorzugsweise im wesentlichen vollständig unterdrückt. Somit wechselt die Strahlungsintensität von der ersten Zeitdauer im infraroten, nicht sichtbaren Wellenlängenbereich und dem sichtbaren Wellenlängenbereich während der zweiten Zeitdauer hin und her. Da bei geeigneten Beleuchtungsquellen die Intensitäten zwar unterschiedlich sind, jedoch nicht zu große Unterschiede aufweisen, wird die Kamera der Beobachtungseinrichtung mit vergleichbar großen Beleuchtungsstärken beaufschlagt. Daher sind keine oder nur geringe Einstellungsänderungen an der Kamera erforderlich, um ein scharfes und gleichzeitig kontrastreiches Bild des Augenhintergrundes zu erzeugen.

Bevorzugt ist dabei weiterhin, daß die Beleuchtungsstärke des Beleuchtungsstrahls vor Eintritt in die Beobachtungseinrichtung, also beispielsweise der Kamera, während der ersten Zeitdauer im wesentlichen gleich groß wie die Beleuchtungsstärke während der zweiten Zeitdauer eingestellt wird. Dieses geschieht durch unterschiedliche elektrische Ansteuerung des Beleuchtungsmittels, also beispielsweise durch unterschiedliche Stromstärken, mit denen beispielsweise eine Halogenlampe beaufschlagt wird. Ebenso ist es möglich, unterschiedlich starke Graufilter zu wählen, um die Beleuchtungsstärkenunterschiede zu realisieren.

Bei einer weiteren bevorzugten Ausgestaltung des erfindungsgemäßen Verfahrens wird während der zweiten Zeitdauer im Strahlengang des Beobachtungsstrahls mit einem Farbfilter der sichtbare Lichtanteil des Beobachtungsstrahls bevorzugt durchgelassen. Dieses dient dazu, daß die Beobachtungseinrichtung, also beispielsweise die Kamera, im wesentlichen nur sichtbares und kein infrarotes Licht erhält. Dadurch wird gewährleistet, daß die Beobachtungseinrichtung ein Bild aufnimmt, das Farben und Kontraste aufweist, wie es eine natürliche Person bei einer direkten Beobachtung des Augenhintergrundes wahrnehmen würde. Zum Ausgleich der optischen Weglänge wird dann vorzugsweise während der ersten Zeitdauer im Strahlengang des Beobachtungsstrahls ein Ausgleichsglas angeordnet. Denn während der ersten Zeitdauer, in der das Auge nur mit infrarotem Licht beaufschlagt wird, wird der zuvor genannte Farbfilter aus dem Strahlengang des Beobachtungsstrahls herausgeschwenkt, um gerade das infrarote Licht zur Beobachtungseinrichtung durchzulassen.

Weiterhin ist es bevorzugt, daß die Länge der zweiten Zeitdauer vorgegeben wird, wobei vorzugsweise die zweite Zeitdauer im Bereich zwischen 0,2 und 2 sec eingestellt wird. Somit kann der Anwender des erfindungsgemäßen Verfahrens den Beobachtungszeitraum des Augenhintergrundes mit sichtbarem Licht begrenzen, so daß ein automatisches Zurückkehren in die Einstellung während der ersten Zeitdauer durchgeführt werden kann, die Aufnahme somit automatisch begonnen und beendet werden kann.

Ein weiterer Vorteil des erfindungsgemäßen Verfahrens besteht darin, daß während der ersten Zeitdauer eine ausreichende Zeit zur Verfügung steht, um die Beleuchtungsstärke in Abhängigkeit vom jeweiligen Auge des Patienten, also der Transparenz des Augenmediums, der Fehlsichtigkeit usw. eingestellt und angepaßt werden kann. Beim Umschalten auf die Beleuchtung mit sichtbarem Licht kann dann die geeignete Beleuchtungsstärke berechnet oder zumindest abgeschätzt werden, um eine ausreichend lichtstarke und kontrastreiche Aufnahme des Augenhintergrundes mit sichtbarem Licht zu erreichen.

Das oben aufgezeigte technische Problem wird erfindungsgemäß auch durch einen Ophthalmoskopen für eine Untersuchung eines Augenhintergrundes eines Patienten mit den Merkmalen des Anspruches 7 gelöst. Es handelt sich um ein Ophthalmoskop mit einer Beleuchtungseinrichtung zum Erzeugen eines Beleuchtungsstrahles, mit einer Optik zum Abbilden des Beleuchtungsstrahls auf das zu untersuchende Auge und zum Abbilden des vom Augenhintergrund reflektierten Beobachtungsstrahls zu einem reellen Zwischenbild und mit einer eine Kamera aufweisenden Beobachtungseinrichtung zum Aufnehmen des Zwischenbildes, bei dem ein sichtbares Licht begrenzender und Infrarotlicht durchlassender erster Filter in den Strahlengang des Beleuchtungsstrahls ein- und ausschwenkbar angeordnet ist. Dabei bedeutet der Begriff "schwenkbar" jede lineare oder nichtlineare Bewegung des Filters, mit dem dieser in den Strahlengang hinein gebracht und aus diesem wieder entfernt werden kann. Insbesondere kann es sich dabei um eine kreisförmige Schwenkbewegung handeln, wie weiter unten erläutert wird.

Weitere Merkmale und Vorteile des erfindungsgemäßen Ophthalmoskopen ergeben sich zum einen durch die oben angegebene Beschreibung des erfindungsgemäßen Verfahrens, das mit Hilfe des genannten Ophthalmoskopen durchgeführt werden kann, und zum anderen aus der nachfolgenden Beschreibung eines Ausführungsbeispiels eines erfindungsgemäßen Ophthalmoskopen. Dabei erfolgt die Beschreibung anhand der beigefügten Zeichnung. In dieser zeigen
- Fig. 1: eine schematische Darstellung des Strahlengangs eines erfindungsgemäßen Ophthalmoskopen,
- Fig. 2: einen Träger zum verschenkbaren Halten der Filter des erfindungsgemäßen Ophthalmoskopen in einer ersten Stellung,
- Fig. 3: den in Fig. 2 dargestellten Träger in einer zweiten Stellung,
- Fig. 4: ein Diagramm mit Transmissionskurven verschiedener Elemente des Ophthalmoskopen und
- Fig. 5: ein Diagramm mit der Beleuchtungsstärke einer Halogenlampe in Abhängigkeit von der angelegten elektrischen Spannung.

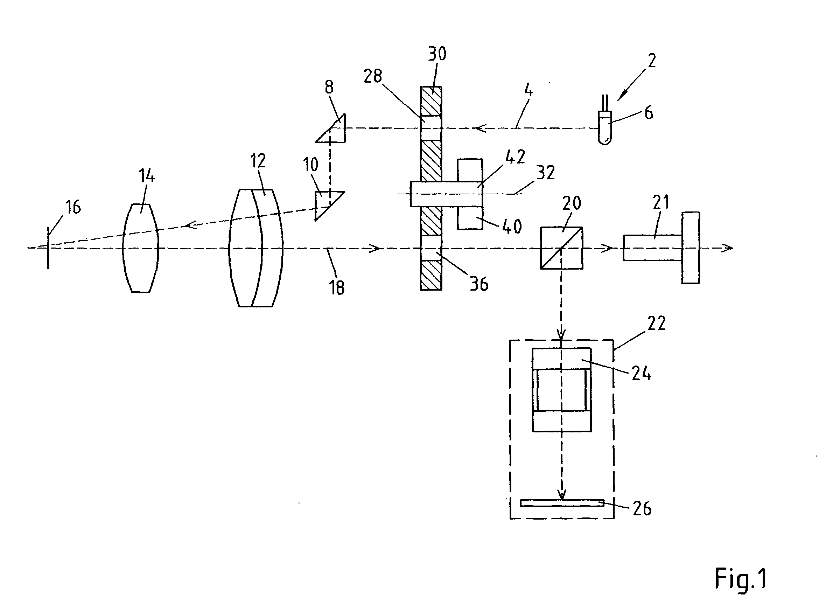

Fig. 1 zeigt den Strahlengang eines erfindungsgemäßen Ophthalmoskopen für eine Untersuchung des Augenhintergrundes eines Patienten.

Eine Beleuchtungseinrichtung 2 zum Erzeugen eines Beleuchtungsstrahles 4 weist eine Halogenlampe 6 auf. Zwei Umlenkprismen 8 und 10 bilden den Beleuchtungsstrahl 4 auf eine Achromatoptik 12 zum Verkürzen des optischen Systems ab. Eine Optik 14, vorliegend nur aus einer Sammellinse bestehend, bildet den Beleuchtungsstrahl 4 auf die schematisch dargestellte Pupille 16 und somit auf den nicht im einzelnen dargestellten Augenhintergrund ab.

Die Linse 14 bildet wiederum das vom Augenhintergrund reflektierte Licht als Beobachtungsstrahls 18 zu einem reellen Zwischenbild ab. Der Beobachtungsstrahl 18 wird von einem Strahlteilerprisma 20 sowohl zu einem Okular 21 zur direkten Beobachtung durch einen Benutzer als auch zu einer eine Kamera 22 aufweisenden Beobachtungseinrichtung gelenkt, mit der das reelle Zwischenbild aufgenommen wird. Die Kamera 22 weist dazu ein Objektiv 24 und einen CCD-Sensor 26 auf, dessen Signale elektrisch weiterverarbeitet werden. Ein Farbfilter ist in der CCD-Kamera 22 nicht vorhanden, so daß das auftreffende Licht ohne weitere Filterung auf den CCD-Sensor 26 trifft.

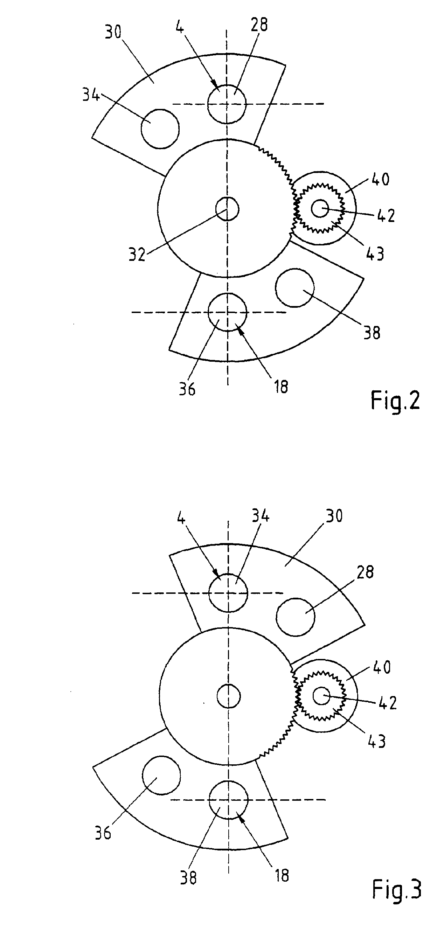

Erfindungsgemäß ist ein sichtbares Licht begrenzender und Infrarotlicht durchlassender erster Filter 28 in den Strahlengang des Beleuchtungsstrahls 4 ein- und ausschwenkbar angeordnet. Dieser ist, wie die Fig. 2 und 3 zeigen, in einem drehbaren Träger 30 angeordnet, der als eine um eine Achse 32 drehbare Segmentrecoss-Scheibe ausgebildet ist. Der Filter 28 kann auch als Infrarotfilter bezeichnet werden.

Darüber hinaus ist ein Infrarotlicht begrenzender und sichtbares Licht durchlassender zweiter Filter 34 in den Strahlengang des Beleuchtungsstrahls 4 ein- und ausschwenkbar angeordnet, indem dieser im Träger 30 beabstandet, jedoch mit im wesentlichen gleichen Abstand zur Achse 32 zum ersten Filter 28 befestigt ist.

In einer ersten Einstellung, die in den Fig. 1 und 2 dargestellt ist, ist der erste Filter 28 im Strahlengang des Beleuchtungsstrahls 4 angeordnet und läßt bevorzugt das infrarote Licht aus dem Beleuchtungsstrahl durch. In einer zweiten, in Fig. 3 dargestellten Einstellung ist der zweite Filter 34 im Strahlengang des Beleuchtungsstrahls 4 angeordnet. Dieses dient einem Durchlassen des sichtbaren Lichtanteils des Beleuchtungsstrahls 4 sowie einem zumindest teilweisen Zurückhalten des infraroten Lichts des Beleuchtungsstrahls 4. Somit können durch Drehen des Trägers 30 um die Drehachse 32 die beiden Filter 28 und 34 abwechselnd in den Strahlengang des Beleuchtungsstrahls 4 eingeschwenkt werden.

Weiterhin zeigen die Fig. 1 und 2, daß im Strahlengang des Beobachtungsstrahls 18 in der ersten Einstellung des Trägers 30 ein Ausgleichsglas 36 zum Ausgleichen der optischen Weglänge angeordnet ist. In der zweiten Einstellung gemäß Fig. 3 ist dagegen ein Farbfilter 38 für eine farbgetreue Darstellung durch die CCD-Kamera 22 angeordnet. Auch hier wird das Verschwenken zwischen der ersten und zweiten Einstellung mittels des um die Achse 32 drehbaren Trägers verwirklicht. Dabei sind der Farbfilter 38 und das Ausgleichsglas 36 am Träger 32 beabstandet zu den Filtern 28 und 34 angeordnet, so daß die Filter paarweise in der jeweiligen Einstellung im Beleuchtungsstrahl 4 und im Beobachtungsstrahl 18 angeordnet sind.

Weiterhin ist in den Fig. 1 bis 3 dargestellt, daß ein steuerbarer Antrieb bestehend aus einem Motor 40 und einer Antriebswelle 42 zum Verstellen des Trägers zwischen der ersten und zweiten Einstellung vorgesehen ist. Dazu greift die Welle 42 über eine Zahnung eines Ritzels 43 am Träger 30 an.

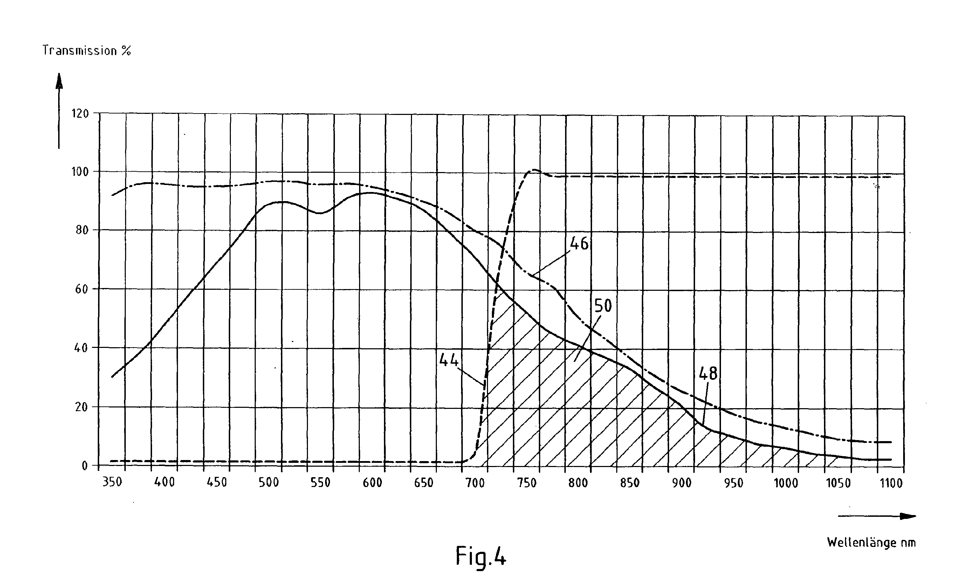

Fig. 4 zeigt die Transmissionskurven der verschiedenen zuvor beschriebenen optischen Komponenten in Abhängigkeit von der Wellenlänge.

Mit 44 ist die Transmissionskurve des ersten Filters 28 bezeichnet, dessen Absorptionskante im Bereich von ca. 715 nm liegt und Licht mit kleinerer Wellenlänge absorbiert, also Licht mit größerer Wellenlänge herausfiltert.

Mit 46 ist die Transmissionskurve des zweiten Filters 34 bezeichnet, dessen Durchlässigkeit im Bereich kleinerer Wellenlängen größer als bei größeren Wellenlängen ist. Dadurch wird erreicht, daß sichtbares Licht bevorzugt durchgelassen wird, während Licht im infraroten Spektralbereich mit größer werdender Wellenlänge zunehmend absorbiert wird. Somit wirken die beiden Filter 28 und 34 in ihrer Funktionsweise einander entgegen.

Mit 48 ist die spektrale Empfindlichtkeit des CCD-Sensors 26 dargestellt, deren Verlauf im Bereich des sichtbaren und infraroten Lichtes weitgehend mit der Transmissionskurve 46 des Lichtes zweiten Filters 34 übereinstimmt.

Das erfindungsgemäße Verfahren wird mit dem beschriebenen Ophthalmoskopen wie folgt durchgeführt. Zunächst wird mit sichtbarer Licht der Beleuchtungsstrahl 4 auf den Augenhintergrund abgebildet und das vom Augenhintergrund als Beobachtungsstrahl 18 reflektierte Licht wird als ein reelles Zwischenbild abgebildet. Dieses reelle Zwischenbild wird dann mit dem Okular 21 beobachtet, um eine Voreinstellung des Ophthalmoskopen auf das Auge durchzuführen. Anschließend der infrarote Betriebsmodus aktiviert und mit der CCD-Kamera 22 wird das reelle Zwischenbild aufgenommen. Damit ist mit aufgeweiteter Pupille ein Einstellen des gesamten Ophthalmoskopen unter abgedunktelten Bedingungen möglich.

Während der ersten Zeitdauer wird also für eine Einstellung der CCD-Kamera 22 der infrarote Lichtanteil des Beleuchtungsstrahls 4 mit Hilfe des ersten Filters 28 bevorzugt durchgelassen und während der zweiten Zeitdauer wird für eine Aufnahme des Augenhintergrundes mit sichtbarem Licht zumindest teilweise der sichtbare Lichtanteil des Beleuchtungsstrahls 4 mit Hilfe des zweiten Filters 34 durchgelassen und auf das Auge abgebildet.

Während der ersten Zeitdauer wird die optische Weglänge im Strahlengang des Beobachtungsstrahls 18 mit einem Ausgleichsglas 36 ausgeglichen und während der zweiten Zeitdauer wird der sichtbare Lichtanteil des Beobachtungsstrahls 18 mit einem Farbfilter 38 herausgefiltert, dessen Transmissionskurve nicht in Fig. 4 dargestellt ist, jedoch im wesentlichen den Wellenlängenbereich zwischen 450 und 700 nm abdeckt.

Fig. 4 zeigt einen weiteren Aspekt der vorliegenden Erfindung. Die auf die Kamera 22 auftreffende Beleuchtungsstärke des Lichtes entspricht in der ersten Einstellung des Trägers 30 der schraffierten Fläche 50. Die Fläche 50 ist dabei von der Empfindlichkeitskurve des CCD-Sensors 26 und der Transmissionskurve des ersten Filters 28 begrenzt. In der zweiten Einstellung des Trägers 30 wird jedoch bei unverändert betriebener Halogenlampe 6 eine Beleuchtungsstärke auf den CCD-Sensor 26 geleitet, der ungefähr dem Dreifachen der Fläche 50 entspricht.

Die Beleuchtungsstärke des Beleuchtungsstrahls vor Eintritt in die CCD-Kamera 22 wird also während der ersten Zeitdauer im Bereich eines Faktors 3 im wesentlichen gleich groß wie während der zweiten Zeitdauer eingestellt. Ein wesentlich besseres Ergebnis der Anpassung der Beleuchtungsstärken wird jedoch erzielt, wenn die Beleuchtungsstärke der Halogenlampe 6 an die erste oder zweite Einstellung angepaßt wird.

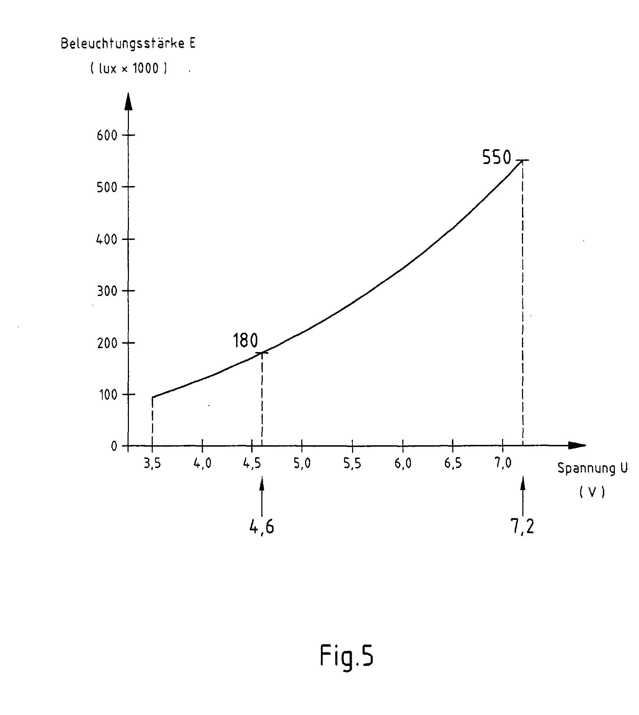

Fig. 5 zeigt ein Diagramm, das die Beleuchtungsstärke der Halogenlampe 6 in Abhängigkeit von der angelegten Spannung zeigt. Wird die angelegte Spännung beispielsweise von der maximalen Spannung von ca. 7,2 Volt auf ca. 4,6 Volt reduziert, so ergibt sich daraus eine Reduzierung der Beleuchtungsstärke um den Faktor 3. Wird also die Halogenlampe 6 in der ersten Einstellung des Trägers 30 mit einer Versorgungsspannung von 7,2 Volt und in der zweiten Einstellung mit 4,6 Volt betrieben, so sind ohne die Verwendung des Farbfilters 38 die Beleuchtungsstärken des auf den CCD-Sensor 26 auftreffenden Beobachtungsstrahls 18 im wesentlichen gleich groß. Mit Verwendung des Farbfilters 38 dagegen braucht die Beleuchtungsstärke nur um den Faktor 2 reduziert werden, da der infrarote Anteil der Beleuchtungsstärke weitgehend vom Farbfilter 38 absorbiert wird und nicht zum CCD-Sensor 22 gelangt.

Somit können die gleichen Kameraeinstellungen in der ersten wie in der zweiten Einstellung beibehalten werden und eine Aufnahme des Augenhintergrundes kann in der zweiten Einstellung ohne zeitliche Verzögerung nach dem Verschwenken des Trägers 30 erfolgen. Denn beispielsweise kann auf ein Nachjustieren der Blendenöffnung der CCD-Kamera 22 verzichtet werden. Somit werden Totzeiten aufgrund einer Nachregelung vermieden und es ist eine schnelle Farbaufzeichnung des Augenhintergrundes mittels der CCD-Kamera 22 ist möglich, bevor sich die Pupille des Auges durch das auftreffende sichtbare Licht verengt hat.

Die zuvor beschriebene Filterbaugruppe des Trägers 30 kann in ansonsten herkömmlichen Ophthalmoskopen integriert werden. In Handgeräten kann das Umschalten durch manuelles Verschenken der Baugruppe erfolgen, während in automatisierten Ophthalmoskopen das Verschenken mit Hilfe des beschriebenen Antriebes 40, 42, 43 erfolgen kann. Die Länge der zweiten Zeitdauer kann dann auch vorwählbar sein, indem mittels eines Potentiometers der Antriebssteuerung die Zeitdauer eingestellt wird, in der das Auge des Patienten mit sichtbarem Licht beaufschlagt wird. Nach Ablauf der zweiten Zeitdauer wird dann mittels des Antriebes 40, 42, 43 der Träger 30 wieder in die erste Einstellung zurück verschwenkt. Ebenso kann bei dieser Ausführungsform das Umschalten der Beleuchtungsstärke der Halogenlampe 6 parallel dazu automatisch erfolgen.

## Patentansprüche

1. Verfahren für eine Untersuchung des Augenhintergrundes eines Patienten in einem abgedunkelten Raum,
- bei dem ein Beleuchtungsstrahl (4) auf den Augenhintergrund abgebildet wird,
- bei dem das vom Augenhintergrund als Beobachtungsstrahl (18) reflektierte Licht als ein reelles Zwischenbild abgebildet wird und
- bei dem das reelle Zwischenbild mit einer Beobachtungseinrichtung (22) aufgenommen wird,
**dadurch gekennzeichnet,**
- **daß** während einer ersten Zeitdauer für eine Einstellung der Beobachtungseinrichtung (22) zumindest teilweise der infrarote Lichtanteil des Beleuchtungsstrahls (4) auf das Auge abgebildet wird und
- **daß** während einer zweiten Zeitdauer für eine Aufnahme des Augenhintergrundes mit sichtbarem Licht zumindest teilweise der sichtbare Lichtanteil des Beleuchtungsstrahls (4) auf das Auge abgebildet wird.

2. Verfahren nach Anspruch 1,
bei dem während der zweiten Zeitdauer der sichtbare Lichtanteil des Beleuchtungsstrahls (4) bevorzugt durchgelassen wird.

3. Verfahren nach Anspruch 1 oder 2,
bei dem die Beleuchtungsstärke (4) des Beleuchtungsstrahls vor Eintritt in die Beobachtungseinrichtung (22) während der ersten Zeitdauer im wesentlichen gleich groß wie während der zweiten Zeitdauer eingestellt wird.

4. Verfahren nach einem der Ansprüche 1 bis 3,
bei dem während der zweiten Zeitdauer der sichtbare Lichtanteil des Beobachtungsstrahls (4) mit einem Farbfilter (38) bevorzugt durchgelassen wird.

5. Verfahren nach Anspruch 4,
bei dem während der ersten Zeitdauer die optische Weglänge im Strahlengang des Beobachtungsstrahls (4) mit einem Ausgleichsglas (36) ausgeglichen wird.

6. Verfahren nach einem der Ansprüche 1 bis 5,
bei dem die Länge der zweiten Zeitdauer vorgegeben wird, vorzugsweise im Bereich zwischen 0,2 und 2 sec eingestellt wird, und bei dem automatisch zwischen der ersten und der zweiten Zeitdauer umgeschaltet wird.

7. Ophthalmoskop für eine Untersuchung des Augenhintergrundes eines Patienten, insbesondere zur Durchführung eines Verfahrens nach einem der Ansprüche 1 bis 6,
- mit einer Beleuchtungseinrichtung (6) zum Erzeugen eines Beleuchtungsstrahles (4),
- mit einer Optik (14) zum Abbilden des Beleuchtungsstrahls auf das zu untersuchende Auge und zum Abbilden des vom Augenhintergrund reflektierten Beobachtungsstrahls (18) zu einem reellen Zwischenbild und
- mit einer eine Kamera (22) aufweisenden Beobachtungseinrichtung zum Aufnehmen des Zwischenbildes,
**dadurch gekennzeichnet,**
- **daß** ein sichtbares Licht begrenzender und Infrarotlicht durchlassender erster Filter (28) in den Strahlengang des Beleuchtungsstrahls (4) ein- und ausschwenkbar angeordnet ist.

8. Ophthalmoskop nach Anspruch 7,
**dadurch gekennzeichnet,**
**daß** ein Infrarotlicht begrenzender und sichtbares Licht durchlassender zweiter Filter (34) in den Strahlengang des Beleuchtungsstrahls (4) ein- und ausschwenkbar angeordnet ist.

9. Ophthalmoskop nach Anspruch 8,
**dadurch gekennzeichnet,**
**daß** in einer ersten Einstellung der erste Filter (28) und in einer zweiten Einstellung der zweite Filter (34) im Strahlengang des Beleuchtungsstrahls (4) angeordnet sind.

10. Ophthalmoskop nach Anspruch 9,
**dadurch gekennzeichnet,**
**daß** im Strahlengang des Beobachtungsstrahls (18) in der ersten Einstellung ein Ausgleichsglas (36) zum Ausgleichen der optischen Weglänge angeordnet ist.

11. Ophthalmoskop nach Anspruch 9 oder 10,
**dadurch gekennzeichnet,**
**daß** im Strahlengang des Beobachtungsstrahls (18) in der zweiten Einstellung ein Farbfilter (38) für eine farbgetreue Darstellung durch die Kamera (22) angeordnet ist.

12. Ophthalmoskop nach einem der Ansprüche 9 bis 11,
**dadurch gekennzeichnet,**
**daß** der erste Filter (28) und der zweite Filter (34) an einem zwischen der ersten und der zweiten Einstellung verstellbaren Träger (30) angeordnet sind.

13. Ophthalmoskop nach Anspruch 12,
**dadurch gekennzeichnet,**
**daß** der Farbfilter (38) und das Ausgleichsglas (36) am Träger (30) beabstandet zu den Filtern (28, 34) angeordnet sind.

14. Ophthalmoskop nach Anspruch 12 oder 13,
**dadurch gekennzeichnet,**
**daß** der Träger (30) als eine um eine Achse (32) drehbare Segmentrecoss-Scheibe ausgebildet ist.

15. Ophthalmoskop nach einem der Ansprüche 12 bis 14,
**dadurch gekennzeichnet,**
**daß** ein, vorzugsweise steuerbarer, Antrieb (40, 42) zum Verstellen des Trägers (30) zwischen der ersten und zweiten Einstellung vorgesehen ist.
